# EUROPEAN PATENT SPECIFICATION

(11) **EP 2 814 495 B1**
(45) Date of publication and mention of the grant of the patent: **07.07.2021**
(21) Application number: 13704767.6
(22) Date of filing: 13.02.2013
(51) Int. Cl.: A61P 1/04, A61K 31/573, A61K 9/28, A61K 9/20, A61K 31/606, A61K 31/395, A61K 31/496

(54) **METHOD FOR TREATING INTESTINAL DISEASES PRESENTING AT LEAST ONE INFLAMMATORY COMPONENT**
METHODE ZUR BEHANDLUNG INTESTINALER ERKRANKUNGEN MIT MINDESTENS EINER ENTZÜNDUNGSKOMPONENTE
PROCÉDÉ DE TRAITEMENT DE MALADIES INTESTINALES PRÉSENTANT AU MOINS UNE COMPOSANTE INFLAMMATOIRE

(30) Priority: 13.02.2012 US 201261598308 P
(43) Date of publication of application: 24.12.2014
(73) Proprietor: Cosmo Technologies Ltd, Dublin 2 (IE); Santarus, Inc., San Diego, CA 92130 (US)
(72) Inventor: MORO, Luigi, I-20020 Lainate (MI) (IT); PROEHL, Gerald Thomas, San Diego, California 92127 (US); WIERENGA, Wendell, Rancho Santa Fe, California 92091 (US); HUANG, Michael Fangching, Cerritos, California 90703 (US); BALLARD II, Emerson David, Carlsbad, California 92009 (US)
(74) Representative: FRKelly
(86) International application number: PCT/EP2013/052838
(87) International publication number: WO 2013/120881

(56) References cited:
- EP-A1- 1 607 087
- CA-A1- 2 274 943
- US-A1- 2012 021 052
- TURSI A ET AL: "Long-term treatment with mesalazine and rifaximin versus rifaximin alone for patients with recurrent attacks of acute diverticulitis of colon", DIGESTIVE AND LIVER DISEASE, W.B. SAUNDERS, GB, vol. 34, no. 7, 1 July 2002 (2002-07-01), pages 510-515, XP009110165, ISSN: 1590-8658, DOI: 10.1016/S1590-8658(02)80110-4
- None

## Description

The present disclosure relates medicaments for use in methods for treating intestinal diseases presenting at least one inflammatory component such as inflammatory bowel disease and/or maintaining remission of intestinal diseases presenting at least one inflammatory component such as inflammatory bowel disease (IBD) using budesonide MMX compositions.

Systemic corticosteroids (SCS) are effective for the short-term induction of remission in active inflammatory bowel disease (IBD), including Ulcerative Colitis (UC) and Crohn's Disease. However, side effects are frequent and the risks associated with long term therapy outweigh the benefits of maintenance treatment. Therefore, alternative agents with fewer side effects are needed. Budesonide, a non-systemic corticosteroid (NSCS) with high first-pass metabolism, combined with MMX® technology, which is designed to deliver active drug to the colon, may be a viable therapeutic option.

Oral dosage forms of budesonide utilizing the MMX® multi-matrix system technology are designed to provide the controlled release and distribution of budesonide throughout the length of the colon. The MMX multi-matrix system technology is described in U.S. Patent Nos. 7,431,943, 7,410,651, RE43,799, and 8,293,273, and U.S. Patent Application Publication No. 2012/0021052, all of which are hereby incorporated by reference in their entireties for all that they disclose. The dosage forms have topical anti-inflammatory activity and due to an extended first pass effect, have less systemic absorption than other corticosteroids.

TURSI A ET AL, reported "Long-term treatment with mesalazine and rifaximin versus rifaximin alone for patients with recurrent attacks of acute diverticulitis of colon", DIGESTIVE AND LIVER DISEASE, vol. 34, no. 7.

EP1607087 relates to a medicinal oral preparation to be delivered to the large intestine comprising a core containing a pharmacologically active ingredient, an inner layer containing one or more cationic polymers and an outer layer containing one or more anionic polymers whereby the core is coated, which is designed so that, in a disintegration test successively consisting of a vertical movement for 2 hours in a first solution of pH 1.2, a vertical movement for 2 hours in a second solution of pH 7.4 and a vertical movement in a third solution of pH 6.4, the average disintegration initiation point and the average disintegration completion point each falls within a period from 35 minutes to 130 minutes after starting the vertical movement in the third solution.

CA2274943 relates to novel compositions for the treatment of inflammatory diseases of the gastrointestinal tract, preferably ulcerative proctitis, ulcerative colitis, Crohn's colitis, radiation proctitis and pouchitis are described. Compositions comprising more than one active agent such as: 5-ASA; a topical steroid such as, budesonide or an R-epimer thereof; and optionally metronidazole, which are more effective than the use of any of these agents alone are also described.

Provided herein are medicaments for use in methods for treating an intestinal disease presenting at least one inflammatory component and/or maintaining remission of an intestinal disease presenting at least one inflammatory component in a patient previously or simultaneously administered a first composition for treatment of said disease.

The medicaments are as defined in the claims. The invention as defined in the claims provides a medicament comprising in combination a first and a second composition for simultaneous, separate or sequential use in treating ulcerative colitis in a patient, said first composition comprising 5-aminosalicylic acid, said second composition being in the form of a single tablet comprising:
(1) a tablet core comprising:
   a) 9 mg of budesonide;
   b) at least one lipophilic excipient;
   c) at least one amphiphilic excipient; and
   d) at least one hydrophilic excipient; and
(2) a coating on said tablet core, said coating comprising a gastro-resistant film;.
wherein the patient is treated with the first composition previously to the second composition; and wherein said patient has a UCDAI score of greater than or equal to 4 prior to said second composition being administered to said patient.

Typically said patient is experiencing an ulcerative colitis flare prior to said second composition being administered to said patient.

Typically the second composition is administered for up to 8 weeks.

Typically after said treatment, said UCDAI score for said patient is less than or equal to 1.

The invention also provides a medicament comprising in combination a first and a second composition for use in treating ulcerative colitis in a patient, said first composition comprising 5-aminosalicylic acid, said second composition being in the form of a single tablet comprising:
1) a tablet core comprising:
   a) 9 mg of budesonide;
   b) at least one lipophilic excipient;
   c) at least one amphiphilic excipient; and
   d) at least one hydrophilic excipient; and
(2) a coating on said tablet core, said coating comprising a gastro-resistant film;
wherein said second composition is administered to said patient once daily for up to 8 weeks or a portion thereof, wherein said ulcerative colitis in said patient is in remission after said treatment, and wherein said patient has a UCDAI score of greater than or equal to 4 prior to said second composition being administered to said patient.

Typically the second composition is administered to said patient for 8 weeks.

Typically, after said treatment, said UCDAI score for said patient is less than or equal to 1.

Typically the patient is experiencing an ulcerative colitis flare prior to said second composition being administered to said patient.

The first and said second compositions may be administered to said patient simultaneously.

The invention also provides a medicament comprising in combination a first and a second composition for use in treating ulcerative colitis in a patient, said patient having been previously administered a composition comprising 5-aminosalicylic acid, wherein said first composition comprises 5-aminosalicylic acid, said second composition being in the form of a single tablet comprising:
1) a tablet core comprising:
   a) 9 mg of budesonide;
   b) at least one lipophilic excipient;
   c) at least one amphiphilic excipient; and
   d) at least one hydrophilic excipient; and
(2) a coating on said tablet core, said coating comprising a gastro-resistant film;
wherein said second composition is administered to said patient once daily for up to 8 weeks or a portion thereof, wherein said ulcerative colitis in said patient is in remission after said treatment, and wherein said patient has a UCDAI score of greater than or equal to 4 prior to said second composition being administered to said patient.

Typically the second composition is administered to said patient for 8 weeks.

Typically after treatment, the UCDAI score for said patient is less than or equal to 1.

Typically the patient is experiencing an ulcerative colitis flare prior to said second composition being administered to said patient.

The first composition and said second composition may be administered to said patient simultaneously.

### DEFINITIONS

Unless otherwise defined, all terms of art, notations and other scientific terminology used herein are intended to have the meanings commonly understood by those of skill in the art to which this disclosure pertains. In some cases, terms with commonly understood meanings are defined herein for clarity and/or for ready reference, and the inclusion of such definitions herein should not necessarily be construed to represent a substantial difference over what is generally understood in the art.

The term "topically delivering" herein refers to the fact that certain compositions may be orally administered to a subject and the active ingredient(s) of the compositions may be released in such ways that the active ingredient(s) may reach the desired organ at a suitable time and extent so that it can exert its typical pharmacological activity, in our case represented by some areas of the gastrointestinal tract. The active ingredient is budesonide. The composition comprises about 9 mg budesonide. The terms "5-ASA" and "mesalamine" used herein refer to 5-aminosalicylic acid, or a pharmaceutically acceptable salt thereof.

The term "remission" used herein generally refers to a reduction in the symptoms of a patient suffering from IBD, such as ulcerative colitis and Crohn's Disease. In some instances, remission may be measured as reduction in an individual patient's UCDAI score, which is well known and can be measured using methods known to those of ordinary skill in the art. UCDAI is a four-component scale (total score of 0 to 12) that encompasses the clinical assessments of stool frequency, rectal bleeding, mucosal appearance and physician's rating of disease activity (score of 0 to 3 for each of the components). In some embodiments, remission is defined as the reduction in a patient's UCDAI score from greater than 1 to a UCDAI score of ≤ 1, with subscores of 0 for rectal bleeding, stool frequency, and mucosal appearance and with a ≥ 1 point reduction in an endoscopy-only score. In other instances, remission may be measured as reduction in Clinical Activity Index (CAI), which is well known and can be measured using methods known to those of ordinary skill in the art. In some embodiments, remission is defined in a patient's CAI score from greater than 4 to a CAI score of no more than 4.

The term "intestinal disease presenting at least one inflammatory component" used herein refers to diseases or conditions known to those of ordinary skill in the art as intestinal diseases which includes inflammation. Examples of inflammatory components are reddening, swelling, exudation, friability, hemorrhage, ulceration. The intestinal disease may be chronic or, in some instances, acute. An example of a chronic intestinal disease presenting at least one inflammatory component is inflammatory bowel disease. An example of an acute intestinal disease presenting at least one inflammatory component is acute diverticulitis.

The terms "IBD" and "inflammatory bowel disease" used herein refer to conditions known to those of ordinary skill in the art as inflammatory bowel disease, such as ulcerative colitis and Crohn's Disease. The inflammatory bowel disease is ulcerative colitis. In some embodiments, the ulcerative colitis may be active mild to medium ulcerative colitis. In any of the embodiments described herein that describe the treatment of and/or maintaining the remission of a chronic intestinal disease are understood to refer to the treatment and/or maintaining the remission of ulcerative colitis, active mild to medium ulcerative colitis.

The term "UC" used herein refers to ulcerative colitis.

The term "matrix" used herein refers to a macroscopically homogeneous structure in all its volume.

The term "matrix-forming excipient" used herein refers to an excipient having chemical-physical characteristics, such as lipophilia, amphiphilia, hydrophilia, or swelling properties as regards hydrophilic matrix, that one of ordinary skill in the art would recognize provide useful properties in preparing and using the compositions described herein. One or more matrix forming-excipients may comprise a matrix. Thus a matrix may comprise one matrix-forming excipient or more than one matrix-forming excipients. For example, a lipophilic matrix can comprise one fatty acid, or it may comprise one or more fatty acids or a fatty acid and a wax. Optionally, other physiologically acceptable excipients (e.g. active substance or lubricants) can be added to the matrix-forming excipient or to a mixture of these compounds to form and confer the desired characteristics to the compositions described herein, such as a lipophilic matrix, and/or an amphiphilic matrix and/or a hydrophilic matrix. One of ordinary skill in the art will recognize that an excipient comprising the compositions described herein may possess more than one characteristic that is useful in preparing and using the compositions disclosed herein. For example, an excipient, such as magnesium stearate or stearic acid, may function as a matrix-forming material and as a lubricant. Furthermore, one of ordinary skill in the art will recognize that there is no particular amount of an excipient that must be present in the compositions disclosed herein in order for such excipient to function as a matrix-forming excipient.

The term "simultaneous, separate or sequential use" used herein refers to administration of the first and second compositions at the same time or in such a manner that the two compositions act in the patient's body at the same time or administration of one composition after the other composition in such a manner to provide a therapeutic effect. In some embodiments the compositions are taken with a meal. In other embodiments, the compositions are taken after a meal, such as 30 minutes or 60 minutes after a meal. In some embodiments, one composition is administered to a patient for a time period followed by administration of the other composition.

### DETAILED DESCRIPTION OF THE DISCLOSURE

Provided are medicaments for use in methods for treating ulcerative colitis in a patient in need of such treatment, said patient having been previously administered a composition comprising 5-aminosalicylic acid, said method comprising administering to said patient a second composition in the form of a single tablet comprising about 9 mg of budesonide.

Also provided is a second composition for treating ulcerative colitis in a patient in need of such treatment, said patient having been previously treated with a composition comprising 5-aminosalicylic acid. In one embodiment, the second composition is in the form of a single tablet comprising about 9 mg of budesonide.

Also provided are methods for treating ulcerative colitis in a patient in need of such treatment, said method comprising administering to said patient a first and a second composition, said first composition comprising 5-aminosalicylic acid and said second composition is in the form of a single tablet comprising about 9 mg of budesonide.

Also provided are a first composition and a second composition for treating ulcerative colitis in a patient in need of such treatment. In one embodiment, the first composition comprises 5-aminosalicylic acid.

Also provided are methods for treating ulcerative colitis in a patient in need of such treatment, said patient having been previously administered a composition comprising 5-aminosalicylic acid, said method comprising administering to said patient a first composition and a second composition, said first composition comprising 5-aminosalicylic acid and said second composition is in the form of a single tablet comprising about 9 mg of budesonide.

Also provided are a first composition and a second composition for treating ulcerative colitis in a patient in need of such treatment, said patient having been previously administered a composition comprising 5-aminosalicylic acid. In one embodiment, the first composition comprises 5-aminosalicylic acid. In one embodiment, the second composition is in the form of a single tablet comprising about 9 mg of budesonide.

Also provided is a medicament comprising in combination a first and a second composition for simultaneous, separate or sequential use in treating an intestinal disease presenting at least one inflammatory component and/or maintaining remission of an intestinal disease presenting at least one inflammatory component in a patient, said first composition comprising at least a compound for treating said disease, said second composition comprising:
(1) a tablet core comprising:
   a) budesonide in an amount effective for treating or maintaining remission of an intestinal disease presenting at least one inflammatory component,
   b) at least one lipophilic excipient;
   c) at least one amphiphilic excipient; and
   d) at least one hydrophilic excipient; and
(2) a coating on said tablet core, said coating comprising a gastro-resistant film.

### First Composition

The first composition may comprise at least one compound chosen from systemic corticosteroids and non-systemic corticosteroids. In some embodiments, first composition comprises a dose of a compound for treating inflammatory bowel disease (IBD), such as, ulcerative colitis or Crohn's Disease. The first composition is a dose of an oral composition comprising 5-ASA, also known as mesalamine, (e.g., ASACOL®, ASACOL® HD, LIALDA®, PENTASA®, and DELZICOL™), or a mesalamine prodrug, for example, sulfasalazine (e.g., AZULFIDINE®), olsalazine (e.g., DIPENTUM®), and balsalazide (COLAZAL®, COLAZIDE®). For mesalamine, a daily dose may be, for example, at least 0.5 g, or at least 0.8 g, or at least 1.6 g, or at least 2 g, or at least 2.4 g, or at least 3 g, or at least 3.2 g, or at least 3.8 g, or at least 4.2 g, or at least 4.6 g, or at least 4.8 g. In some embodiments, the first composition comprises at least 2.4 g mesalamine. In other embodiments, the first composition comprises about 2.4 g mesalamine. For sulfasalazine, a dose may be, for example, at least 2 g, or at least 4 g. In an add-on therapy in which the patient has been previously treated for IBD with a first composition, the patient may have been treated with a first composition for a period of at least 1 week, at least 2 weeks, at least 4 weeks, at least 8 weeks, at least 16 weeks, or at least 1 year, or any time period desirable for inducing or maintaining remission of IBD.

In an add-on therapy in which the patient has been previously treated for an intestinal disease presenting at least one inflammatory component with a first composition, the patient may have been treated with a first composition for a period of at least 1 week, at least 2 weeks, at least 4 weeks, at least 8 weeks, at least 16 weeks, or at least 1 year, or any time period desirable for inducing or maintaining remission of an intestinal disease presenting at least one inflammatory component.

### Second Composition

The second composition may comprise a tablet comprising budesonide. In one embodiment, the tablet may comprise budesonide in an amount effective for treating inflammatory bowel disease and/or maintaining remission of inflammatory bowel disease, 9 mg of budesonide. The tablet may comprise budesonide, at least one lipophilic excipient, at least one amphiphilic excipient, and at least one hydrophilic excipient.

The second composition may comprise a tablet core and a coating on the tablet core. The tablet core may comprise budesonide in an amount effective for treating inflammatory bowel disease and/or maintaining remission of inflammatory bowel disease, at least one lipophilic excipient, at least one amphiphilic excipient, and at least one hydrophilic excipient.

The second composition comprises 9 mg of budesonide, , at least one lipophilic excipient, at least one amphiphilic excipient, and at least one hydrophilic excipient. In some embodiments, the tablet may be a multi-matrix tablet. In one embodiment, at least one lipophilic excipient is a lipophilic matrix-forming excipient. In another embodiment, at least one amphiphilic excipient is an amphiphilic matrix-forming excipient. In another embodiment, at least one hydrophilic excipient is a hydrophilic matrix-forming excipient.

The second composition comprises about 9 mg budesonide as a single dose or as a divided dose. The second composition may comprise a tablet core and a coating on the tablet core. The tablet core may comprise budesonide in an amount effective for treating an intestinal disease presenting at least one inflammatory component and/or maintaining remission of an intestinal disease presenting at least one inflammatory component, at least one lipophilic excipient, at least one amphiphilic excipient, and at least one hydrophilic excipient. In some embodiments, the tablet core may be a multi-matrix tablet core. In one embodiment, at least one lipophilic excipient is a lipophilic matrix-forming excipient. In another embodiment, at least one amphiphilic excipient is an amphiphilic matrix-forming excipient. In another embodiment, at least one hydrophilic excipient is a hydrophilic matrix-forming excipient.

The tablet core comprises about 9 mg budesonide. In some embodiments, the at least one lipophilic excipient or the at least one lipophilic matrix-forming excipient may be chosen from unsaturated or hydrogenated alcohols or fatty acids, salts, esters or amides thereof, fatty acids mono-, di- or triglycerides, the polyethoxylated derivatives thereof, waxes, ceramides, cholesterol derivatives or mixtures thereof. In some embodiments, the at least one lipophilic excipient or the at least one lipophilic matrix-forming excipient may have a melting point within the range of 40 to 90°C, for example, from 50 to 80°C, or from 60 to 70°C. In some embodiments, the at least one lipophilic excipient or the at least one lipophilic matrix-forming excipient may be selected from saturated or unsaturated fatty acids, such as palmitic, stearic, myristic, lauric, laurylic, or oleic acids or mixtures thereof with other fatty acids with shorter chain. In some embodiments, the at least one lipophilic excipient or the at least one lipophilic matrix-forming excipient may be stearic acid. If desired, a fatty acid calcium salt may be incorporated in the at least one lipophilic excipient or in the at least one lipophilic matrix-forming excipient. In some embodiments and if desired, a physiologically acceptable salt of a fatty acid, such as sodium, calcium or magnesium salts, may be incorporated in the at least one lipophilic excipient or in the at least one lipophilic matrix-forming excipient.

In some embodiments, the at least one amphiphilic excipient or the at least one amphiphilic matrix-forming excipient may be chosen from polar lipids of type I or II (lecithin, phosphatidylcholine, phosphatidylethanolamine), ceramides, glycol alkyl ethers such as diethylene glycol monomethyl ether (e.g. TRANSCUTOL®). In some embodiments, the at least one amphiphilic excipient or the at least one amphiphilic matrix-forming excipient may be lecithin.

In some embodiments, the at least one hydrophilic excipient or the at least one hydrophilic matrix-forming excipient may be chosen from hydrogels, i.e. substances which when passing from the dry state to the hydrated one, undergo the so-called "macromolecular relaxation," namely a remarkable increase in mass and weight following the coordination of a large number of water molecules by the polar groups present in the polymeric chains of the excipients themselves. Examples of hydrogels include, but are not limited to, acrylic or methacrylic acid polymers or copolymers, alkylvinyl polymers, hydroxyalkyl celluloses, carboxyalkyl celluloses, polysaccharides, dextrins, pectins, starches and derivatives, natural or synthetic gums, and alginic acid. In some embodiments, the at least one hydrophilic excipient or the at least one hydrophilic matrix-forming excipient may be chosen from polyalcohols such as xylitol, maltitol and mannitol. In some embodiments, the at least one hydrophilic excipient or the at least one hydrophilic matrix-forming excipient may be chosen from hydroxyalkyl celluloses. In some embodiments, the at least one hydrophilic excipient or the at least one hydrophilic matrix-forming excipient may be hydroxypropylcellulose, hydroxypropylmethylcellulose, an alkylcellulose derivative, or a hydroxyalkylcellulose derivative or a mixture thereof. In some embodiments, the hydrophilic excipient or the hydrophilic matrix-forming excipient may be hydroxypropylcellulose, hydroxypropylmethylcellulose, or a mixture thereof. In some embodiments, the hydrophilic excipient or the hydrophilic matrix-forming excipient may be hydroxypropylcellulose. In some embodiments, the hydrophilic excipient or the hydrophilic matrix-forming excipient may be hydroxypropylmethylcellulose. In some embodiments, the hydrophilic excipient is not a natural or synthetic gum. In some embodiments, the hydrophilic excipient is not a natural gum. In some embodiments, the hydrophilic excipient is not a synthetic gum. In some embodiments, the hydrophilic excipient is not guar gum.

The coating on said tablet core may comprise a gastro-resistant film. The gastro-resistant film may be chosen from acrylic and/or methacrylic acids polymers or copolymers (EUDRAGIT® R or S/L) or cellulose derivatives, such as cellulose acetophthalate/s. In some embodiments, the gastro-resistant film may be chosen from at least one methacrylic acid polymer or copolymer or a mixture of methacrylic acid polymers or copolymers. In some embodiments, the gastro-resistant film may be a mixture of acrylic and/or methacrylic acid copolymers type A and/or type B (for example, EUDRAGIT® S100 and/or EUDRAGIT® L100 or EUDRAGIT® RS). In some embodiments, the gastro-resistant film may be methacrylic acid copolymer types A and B.

In some embodiments, the second composition may further comprise at least one other pharmaceutically acceptable excipient. In some embodiments, the at least other one pharmaceutically acceptable excipient may be chosen from microcrystalline cellulose, lactose, silicon dioxide (silica), magnesium stearate, talc, triethylcitrate, and titanium dioxide.

In some embodiments, the second composition may comprise about 9 mg budesonide, stearic acid, lecithin, microcrystalline cellulose, hydroxypropylcellulose, lactose, silicon dioxide, magnesium stearate, methacrylic acid copolymer types A and B, talc, triethylcitrate, and titanium dioxide.

Non-limiting exemplary embodiments of the second composition and non-limiting exemplary embodiments for preparing the second composition may be found in U.S. Patent Nos. 7,431,943, 7,410,651, RE43,799, 8,293,273, and U.S. Patent Application Publication No. 2012/0021052.,

The second composition may be administered to a patient who is in need of treatment of mild to moderate IBD. In some embodiments, the patient may be in need of maintaining remission of IBD. The IBD may be mild to moderate active ulcerative colitis (also referred to as active mild to moderate ulcerative colitis). In some embodiments, the second composition comprises budesonide and is administered to patients for the induction of remission in patients with active, mild to moderate ulcerative colitis. The second composition comprises about 9 mg budesonideThe second composition may be administered to a patient who is in need of treatment of an intestinal disease presenting at least one inflammatory component. In some embodiments, the patient may be in need of maintaining remission of intestinal disease presenting at least one inflammatory component. The disease presenting at least one inflammatory component is a chronic disease or an acute or acute phase is IBD. The IBD is ulcerative colitis.or The IBD may be active mild to moderate active ulcerative colitis (also referred to as active mild to moderate ulcerative colitis). In some embodiments, the second composition comprises budesonide and is administered to patients for the induction of remission in patients with active, mild to moderate ulcerative colitis. In another embodiment, the second composition comprises budesonide and is administered to patients for the induction of remission in patients with active, mild to moderate ulcerative colitis with an inadequate response to the administration of a first composition comprising mesalamine. The second composition comprises about 9 mg budesonide.

In some embodiments, the second composition may be administered for a time period of at least 1 week, at least 2 weeks, at least 4 weeks, at least 8 weeks, at least 16 weeks, at least 6 months, at least 9 months, at least 12 months, or at least 24 months, or any time period desirable for inducing or maintain remission of IBD. In one embodiment, the IBD is ulcerative colitis. In some embodiments, the second composition may be administered for a time period of at least 1 week, at least 2 weeks, at least 4 weeks, at least 8 weeks, at least 16 weeks, at least 6 months, at least 9 months, at least 12 months, or at least 24 months, or any time period desirable for inducing or maintain remission of an intestinal disease presenting at least one inflammatory component.

In another embodiment, the second composition comprises 9 mg of budesonide and is administered to a patient once daily in the morning with or without food for up to 8 weeks. In another embodiment, the second composition comprises a delayed and extended release tablet comprising about 9 mg of budesonide and is administered to a patient once daily in the morning with or without food for up to 8 weeks. In some embodiments, the amount of budesonide in the second composition is 9 mg.

In some embodiments, the second composition comprises tablets that should be swallowed whole with water and not chewed, crushed or broken. In another embodiment, the patients to whom the second composition is administered should be advised to avoid the consumption of grapefruit juice for the duration of the time during which they are being administered the second composition.

In some embodiments, the first composition comprises about 0.5 g to about 4.8 g 5-aminosalicylic acid and the second composition comprises about 9 mg budesonide, such as about 0.8 g 5-aminosalicyclic acid and about 9 mg budesonide, or about 2 g 5-aminosalicyclic acid and about 9 mg budesonide, or about 2.4 g 5-aminosalicyclic acid and about 9 mg budesonide, or about 4.2 g 5-aminosalicyclic acid and about 9 mg budesonide.

### EXAMPLES

The examples described below may be modified using methods known to those of ordinary skill in the art to prepare tablets, each of which about 9 mg budesonide.

Unless otherwise indicated, the dissolution test described herein is performed by introducing individual tablets in a vessel containing from 500 to 1000 ml of a buffered solution set to pH 7.2, so that the pH conditions of large intestine should be reproduced. To simulate the human body conditions, the test is carried out at a temperature of 37° C ± 2° C and at predetermined time periods samples of the dissolution medium are withdrawn to detect the percentage of active ingredient dissolved over time.

### EXAMPLE 1

### Budesonide MMX® Composition

2.7 kg of budesonide, 3.0 kg of lecithin (amphiphilic excipient) and 3.0 kg of stearic acid (lipophilic excipient) are mixed after sieving till a homogeneous mixture is obtained. Then 39.0 kg of inert, functional excipients and 9.0 kg of low viscosity hydroxypropylcellulose (binder) are added and mixed for 10 minutes before adding purified water and kneading to a suitable consistency. Then pass the granulate through a rotating granulator equipped with the suitable screen and transfer the granulate to the fluid bed drier to lower the residual moisture content under 3%. After a new sieving of the dried granulate, 9.0 kg of hydroxypropylcellulose (hydrophilic excipient) and the suitable amount of functional excipients (in particular, microcrystalline cellulose, lactose and silicon dioxide) are added, and, after 15 minutes of mixing, magnesium stearate in a suitable quantity to act as lubricant is added.

After a final blending, tablets of around 300 mg of unitary weight are generated.

The core are then coated with a suspension obtained introducing into a stainless steel container 5.8 kg of Eudragit™ (methacrylate copolymers), 0.6 kg of triethylcitrate and 3.0 kg of dyes and talc, using alcohol as solvent.

The mean dissolution percentage (as average of six or more tablets) obtained with this tablet formulation were around 10-20% at second hour sampling, in the range 25% to 65% at fourth hour and a dissolution greater than 80% was achieved at 8^{th} hour sampling.

### EXAMPLE 2

### Budesonide MMX® Composition

| | Component | mg/tablet |
|---|---|---|
| Tablet | | |
| | Budesonide | 9.0 |
| | Stearic Acid (lipophilic matrix forming materials) | 10.0 |
| | Lecithin (amphiphilic matrix forming material) | 10.0 |
| | Microcrystalline cellulose | 156.0 |
| | Hydroxypropylcellulose | 60.0 |
| | Lactose monohydrate | 50.0 |
| | Silica, colloidal hydrated | 2.0 |
| | Magnesium stearate | 3.0 |

| Coating materials | | |
|---|---|---|
| | Eudragit L100 (acrylic and methacrylic copolymer) | 14.0 |
| Eudragit S100 (acrylic and methacrylic copolymer) | | 12.0 |
| Talc | | 7.9 |
| Titanium dioxide | | 4.5 |
| Triethylcitrate | | 1.6 |
| Alcohol | | q.s. |

The coating of industrial scale tablets of batch MV084 contained 8.0 mg of Eudragit L100 and 8.0 mg of Eudragit S100 (instead of 14.0 mg and 12.0 mg, respectively) with an individual weight of about 330 mg.

According to the present disclosure, coated tablets individually weighing about 330 mg or about 340 mg are obtained.

The above described dissolution test is performed on the tablets of Example 2. The results are the following (indicated as average value):

| | |
|---|---|
| - after 2 hours at pH 1 | resistant (<5%) |
| - after 1 hour at pH 6.4 | resistant (<5%) |
| - after 2 hours at pH 7.2 | 15% |
| - after 4 hours at pH 7.2 | 37% |
| - after 8 hours at pH 7.2 | 91% |

### EXAMPLE 2A

### Budesonide MMX® Composition

Tablets comprising 9 mg of budesonide and having the following composition were prepared having an individual weight of about 330 mg.

| | Component | mg/tablet |
|---|---|---|
| Tablet | | |
| | Budesonide | 9.0 |
| | Stearic Acid (lipophilic matrix forming materials) | 10.0 |
| | Lecithin (amphiphilic matrix forming material) | 10.0 |
| | Microcrystalline cellulose | 156.0 |
| | Hydroxypropylcellulose | 60.0 |
| | Lactose monohydrate | 50.0 |
| | Silica, colloidal hydrated | 2.0 |
| | Magnesium stearate | 3.0 |

| Coating materials | | |
|---|---|---|
| | Eudragit L100 (methacrylic copolymer, Type A) | 8.0 |
| | Eudragit S100 (methacrylic copolymer, Type B) | 8.0 |
| | Talc | 7.9 |
| | Titanium dioxide | 4.5 |
| | Triethylcitrate | 1.6 |
| | Alcohol | q.s. |

Coated tablets individually weighing about 330 mg were obtained.

### REFERENCE EXAMPLE 2B

### Budesonide MMX® Composition

Tablets comprising 6 mg of budesonide and having the following composition were prepared having an individual weight of about 330 mg.

| | Component | mg/tablet |
|---|---|---|
| Tablet | | |
| | Budesonide | 6.0 |
| | Stearic Acid (lipophilic matrix forming materials) | 10.0 |
| | Lecithin (amphiphilic matrix forming material) | 10.0 |
| | Microcrystalline cellulose | 156.0 |
| | Hydroxypropylcellulose | 60.0 |
| | Lactose monohydrate | 53.0 |
| | Silicon dioxide | 2.0 |
| | Magnesium stearate | 3.0 |

| Coating materials | | |
|---|---|---|
| Eudragit L100 (acrylic and methacrylic copolymer) | | 8.0 |
| Eudragit S100 (acrylic and methacrylic copolymer) | | 8.0 |
| Talc | | 7.9 |
| Titanium dioxide | | 4.5 |
| Triethylcitrate | | 1.6 |
| Alcohol | | q.s. |

Coated tablets individually weighing about 330 mg were obtained.

### EXAMPLE 3

### Budesonide MMX® Composition

Budesonide (3.0 kg) is mixed with soybean Lecithin (5.0 kg) until a homogeneous mixture is obtained. Then carnauba wax (2.0 kg) and stearic acid (2.0 kg) sieved through a fine screen are added. After mixing, the powders are added with other functional excipients and kneaded with a binder solution obtained by dissolving medium viscosity polyvinylpirrolidone in water. After drying in a fluid bed and milling throughout a suitable screen, hydroxypropylmethylcellulose (35.0 kg) and other excipients, including magnesium stearate as lubricant, in a suitable quantity are added and the mixture is blended till a homogeneous powder dispersion is obtained.

The powder mixture is subjected to compression in a rotating tabletting machine and the tablets so obtained are coated in a pan coat with a gastroresistant composition containing Eudragit™, plasticizers, dyes and pigments.

According to the present example, coated tablets individually weighing around 105 mg are obtained.

The results of the above described dissolution test are the following (indicated as average value of at least six tablets):

| | |
|---|---|
| - after 2 hours at pH 1 | resistant (<5%) |
| - after 1 hour at pH 6.4 | resistant (<5%) |
| - after 2 hours at pH 7.2 | 9% |
| - after 4 hours at pH 7.2 | 28% |
| - after 8 hours at pH 7.2 | 86% |

### EXAMPLE 4

### Budesonide MMX® Composition

50 g of diethylene glycol monoethyl ether are homogeneously distributed on 500 g of microcrystalline cellulose; then 100 g of budesonide are added, mixing to complete homogenization. This mix is further added with 400 g of budesonide, then dispersed in a blender containing 100 g of carnauba wax and 100 g of stearic acid preheated at a temperature of 60° C. After kneading for 5 minutes, the mixture is cooled to room temperature and extruded in granules of size below 1 mm. A suitable mixer is loaded with the matrix granules prepared as above and the following amounts of hydrophilic excipients: 1500 g of hydroxypropyl methylcellulose and 500 g of Policarbophil™ are added. The components are mixed until homogeneous dispersion of the matrices, then added with 2450 g of microcrystalline cellulose, 400 g of lactose, 100 g of colloidal silica and 50 g of magnesium stearate. After further 5 minute mixing, the mix is tableted to unitary weight of 250 mg/tablet.

Tablets are then subjected to coating using a suspension containing polyacrylate and polymethacrilate copolymers in addition to dyes, plasticizers and colouring agents in solvent (ethyl alcohol).

The results of the dissolution test performed on these coated tablets are the following (indicated as average value of at least six tablets):

| | |
|---|---|
| - after 2 hours at pH 1 | resistant (<5%) |
| - after 1 hour at pH 6.4 | resistant (<5%) |
| - after 2 hours at pH 7.2 | 11% |
| - after 4 hours at pH 7.2 | 32% |
| - after 8 hours at pH 7.2 | 76% |

### EXAMPLE 5

### Budesonide MMX® for the Maintenance of Remission in Patients with Ulcerative Colitis (UC)

Budesonide MMX (budesonide MMX) 9 mg tablets having the composition as in Example 2A administered once daily has been shown to be well tolerated and effective at inducing both clinical and endoscopic remission with symptom resolution in patients with mild-to-moderate UC after 8 weeks of therapy. To evaluate the potential efficacy and safety of long term therapy on the maintenance of remission with budesonide MMX, a Phase III, 12-month placebo (placebo)-controlled safety and extended use study was conducted.

### Efficacy Analysis

To evaluate the efficacy of budesonide MMX 6 mg tablets having the composition as in Example 2B, for up to 12 months for the maintenance of clinical remission (i.e. rectal bleeding (RB)=0 and stool frequency (SF)=0 based on UCDAI score), 122 patients, who were in clinical and endoscopic remission after two Phase 3 studies or an Open Label study, were randomized (1:1) to budesonide MMX 6 mg QD or placebo. Exploratory efficacy evaluation included the portion of patients in clinical remission (primary endpoint) after 1, 3, 6, 9, 12 months and/or End of Study/Early Withdrawal Visit. Secondary endpoint was time to clinical relapse (the time in days to recurrence of RB and/or SF ≥ 1-2 stools/day above normal for the patient).

Potentially due to insufficient statistical power, efficacy analysis did not show significant difference between budesonide MMX 6 mg and placebo for the primary endpoint (p value was greater than 0.05). The probability of clinical relapse at 12 months was reduced with budesonide MMX 6 mg compared to placebo. Moreover, the median time to clinical relapse was longer and statistically significant in intended-to-treat (ITT) population in the budesonide MMX group compared to placebo (Table 1). Treatment Emergent adverse events (AEs) were similar between treatment groups (21.0%) and placebo (21.3%).

In this 12 month extended use study, the probability of clinical relapse was less likely with budesonide MMX 6 mg QD over placebo. Budesonide MMX 6 mg prolonged the median time to clinical relapse vs. placebo. Therefore, extended use of budesonide-MMX 6 mg is an option for long term therapy for the maintenance of remission in UC patients.

**TABLE 1**

| | Efficacy Evaluable Analysis* | | Intended-to-Treat Analysis** | |
|---|---|---|---|---|
| | budesonide MMX 6 mg | placebo | budesonide MMX 6 mg | placebo |
| Probability of Clinical Relapse | 40.4% | 60.1% | 40.9% | 59.7% |
| p-value | 0.0546 | | 0.0224 | |
| Median Time to Clinical Relapse | > 1 yr, never reach | 182 days | > 1 yr, never reach | 181 days |

| | | | | |
|---|---|---|---|---|
| *All randomized patients who received at least one dose of a study drug, but excluding patients who were not in remission at the end of the parent study, who had normal histology at baseline in the parent study, were enrolled at a site with major GCP violations in the parent study, were withdrawn from the study because of insufficient bone density ** All randomized patients who received at least one dose of a study drug | | | | |

### Safety Analysis

Safety analysis included 123 Patients, who were in clinical and endoscopic remission after two Phase 3 studies or an Open Label study with budesonide MMX 9 mg tablets having the composition as in Example 2A. All patients who received at least one dose of budesonide MMX 6 mg tablets having the composition as in Example 2B, or placebo were included in the safety analysis. The patients were randomized (1:1) to budesonide MMX 6 mg QD or placebo. All patients Safety assessment was conducted at baseline, 1, 3, 6, 9, 12 months of treatment and/or End of Study/Early Withdrawal Visit.

As shown in Table 2, potential glucocorticoid effects occurred at similar rates between treatment groups. Treatment-related AEs (budesonide MMX 6 mg (21.0%) and placebo (21.3%)) were similar between groups. Mean morning cortisol levels remained within the normal range for all treatment visits. SAEs were infrequently reported (one patient (1.6%) in each group) and none were related to study drug. There were no deaths or life-threatening events during the study. This extended use study showed that the rates of potential glucocorticoid effects, TEAEs, and TREAs were similar to placebo for long term maintenance of remission therapy. Thus, budesonide MMX 6 mg administered once daily for up to 12 months was well tolerated in patients with UC and with a comparable safety profile to placebo.

**TABLE 2**

| **MEDRA Preferred Term** | **Placebo** | **MMX 6 mg** |
|---|---|---|
| | **n (%)** | **n (%)** |
| **Potential Glucocorticoid Effects** | **N=61** | **N=62** |
| Overall incidence | 7 (11.5) | 9 (14.5) |
| Moon face | 3 (4.9) | 3 (4.8) |
| Striae rubrae | 0 | 0 |
| Flushing | 1 (1.6) | 1 (1.6) |
| Fluid retention | 1 (1.6) | 1 (1.6) |
| Mood changes | 2 (3.3) | 4 (6.5) |
| Sleep changes | 3 (4.9) | 3 (4.8) |
| Insomnia | 4 (6.6) | 4 (6.5) |
| Acne | 0 | 3 (4.8) |
| Hirsutism | 0 | 3 (4.8) |

| **Treatment Emergent AEs (TEAE) ≥ 5%** | **N= 61** | **N= 62** |
|---|---|---|
| Patients with any TEAEs | 44. (72.1) | 40 (64.5) |
| Colitis ulcerative | 16 (26.2) | 11 (17.7) |
| Abdominal pain | 5 (8.2) | 6 (9.7) |
| Headache | 2 (3.3) | 4 (6.5) |
| Osteopenia | 5 (8.2) | 1 (1.6) |
| Frequent bowel movements | 1 (1.6) | 4 (6.5) |
| Haematochezia | 1 (1.6) | 4 (6.5) |
| Constipation | 0 | 4 (6.5) |

| **Treatment-related AEs (TRAE) ≥ 2%** | **N=61** | **N=62** |
|---|---|---|
| Patients With Any Treatment-related AE | 13 (21.3) | 13 (21.0) |
| Colitis Ulcerative | 3 (4.9) | 4 (6.5) |
| Osteopenia | 4 (6.6) | 1 (1.6) |
| Cushingoid | 1 (1.6) | 3 (4.8) |
| Abdominal Pain | 1 (1.6) | 2 (3.2) |
| Flushing | 1 (1.6) | 2 (3.2) |
| Hirsutism | 0 | 2 (3.2) |

### Effect of Extended Use of budesonide MMX on Bone Marrow Density

Although systemic corticosteroids (SCS) are effective for the short-term induction of remission in active UC, long term use can lead to unacceptable side effects such as a reduction in bone mineral density (BMD). Budesonide is a non-systemic corticosteroid (NSCS), and budesonide MMX (budesonide MMX) 9 mg tablets having the composition as in Example 2A, administered once daily has been shown to be well tolerated in patients with mild-to-moderate UC after 8 weeks of therapy. In this extended use study, BMD was evaluated after long term therapy with budesonide MMX 6 mg tablets having the composition as in Example 2B, over 12 months.

BMD was assessed at baseline and 12 months or the End of Study/Early Withdrawal Visit by dual-energy X-ray absorptiometry (DEXA) scan or alternative radiological methods if DEXA was not available. Patients were required to receive therapy for at least 6 months to be included in the BMD evaluation.

Baseline BMD scans were obtained from the 123 patients at the start of the long term study. 74 patients were eligible for inclusion in the BMD analysis (on drug for 6 months) (N=39; placebo and N=35; budesonide MMX 6 mg). At the end of the study, 66 patients had both baseline and end-of-study BMD scan for comparison.

As shown in Table 3, normal BMD was observed in 74% (placebo) and 78% (budesonide MMX 6 mg) at 12 months. BMD remained unchanged compared to baseline in 77% (placebo) and 86% (budesonide MMX 6 mg). Worsening of BMD was observed in 3 pts (7.7%) in placebo and 2 pts (5.7%) in budesonide MMX 6 mg. (Table 3). Overall, there were no clinically important differences between placebo and budesonide MMX 6 mg at the end of the study. There were no bone fractures reported by either group during the study. Thus, the effect of extended use of budesonide MMX 6 mg on bone mineral density appears to be comparable to placebo.

**TABLE 3**

| **Bone Mineral Density Evaluation at 12 months** | **Placebo** | **MMX 6 mg** |
|---|---|---|
| | **n (%)** | **n (%)** |
| **All scan methods** | **39** | **35** |
| Normal | 29 (74.4) | 27 (77.1) |
| Abnormal | 5 (12.8) | 5 (14.3) |
| Not Done | 2 (5.1) | 0 |
| Missing | 3 (7.7) | 3 (8.6) |

| **DEXA Scan** | **35** | **29** |
|---|---|---|
| Normal (T-score > -1) | 27 (77.1) | 21 (72.4) |
| Abnormal (T-score ≤ -1) | 5 (14.3) | 5 (17.2) |
| Osteopenia (T-score > -2.5 and ≤ -1) | 5 (100.0) | 5 (100.0) |
| Osteoporosis (T-score ≤ -2.5) | 0 | 0 |
| Missing | 3 (8.6) | 3 (10.3) |

| **Other Scan methods** | **4** | **6** |
|---|---|---|
| Normal | 2 (50.0) | 6 (100.0) |
| Abnormal | 0 | 0 |
| Not Done | 2 (50.0) | 0 |

| **Change from baseline to last visit** | **39** | **35** |
|---|---|---|
| Improved | 1 (2.6) | 0 |
| Unmodified | 30 (76.9) | 30 (85.7) |
| Worsened | 3 (7.7) | 2 (5.7) |
| Missing | 5 (12.8) | 3 (8.6) |

### Effect of Extended Use of budesonide MMX on the Hypothalamic-Pituitary-Adrenal (HPA) Axis

Although systemic corticosteroids (SCS) are effective for the short-term induction of remission in active ulcerative colitis (UC), long term use can lead to suppression of adrenal gland function resulting in reduction of circulating cortisol. Significant long term adrenal suppression may cause an adrenal crisis during acute illness, trauma, surgery, or other stress. In the 12 month study, cortisol levels and adrenocorticotropic hormone stimulation tests were evaluated during extended therapy with budesonide MMX 6 mg tablets having the composition as in Example 2B.

To assess adrenal gland function, morning plasma cortisol (MPC) levels and safety assessments were conducted at baseline, 1, 3, 6, 9, and 12 months and/or End of Study/Early Withdrawal Visit. adrenocorticotropic hormone stimulation test was performed at 12 months and/or End of Study/Early Withdrawal Visit.

At each visit, mean MPC values remained within normal limits (138 to 690 nmol/L) for both treatment groups. As shown in Table 4, a slight reduction of MPC was observed within 1 month of budesonide MMX therapy but remained within normal limits (138 to 690 nmol/L) and showed no further decline over 12 months. Mean morning cortisol levels at month 12 (nmol/L ± SD) were 287.4 ± 160.9 for budesonide MMX 6 mg and 359.7 ± 113.4 for placebo, which were within normal limits. At Month 12/Final Visit, adrenocorticotropic hormone stimulation test showed more patients had normal values in placebo [82% (28/34)] than the budesonide MMX 6 mg group [70% (23/33)] (p=0.22). This is similar to other budesonide formulations approved for the treatment of IBD, as reported in the literature. Potential glucocorticoid effects (11.5% placebo and 14.5% budesonide MMX) and treatment-related AE (21.3% placebo and 21.0% budesonide MMX) did not differ between treatment groups. No clinically significant trends in AE (including acute adrenal failure) were observed in the study. Thus, budesonide MMX 6 mg was well tolerated and the glucocorticoid effects are compared to placebo.

**TABLE 4**

| | Placebo | MMX 6 mg |
|---|---|---|
| Baseline, N | 59 | 59 |
| Mean ± SD (nmol/L) | 287.3 ± 153.5 | 244.6 ± 141.9 |

| Month 1, N | 45 | 49 |
|---|---|---|
| Mean ± SD (nmol/L) | 287.3 ± 98.2 | 224.7 ± 132.2 |

| Month 3, N | 40 | 45 |
|---|---|---|
| Mean ± SD (nmol/L) | 309.6 ± 96.8 | 223.4 ± 158.1 |

| Month 6, N | 35 | 31 |
|---|---|---|
| Mean ± SD (nmol/L) | 337.3 ± 124.8 | 290.6 ± 134.7 |

| Month 9, N | 30 | 29 |
|---|---|---|
| Mean ± SD (nmol/L) | 345.6 ± 116.4 | 284.1 ± 108.1 |

| Month 12- completers^{a}, N | 21 | 22 |
|---|---|---|
| Mean ± SD (nmol/L) | 348.0 ± 121.7 | 310.3 ± 141.7 |
| Changes from baseline | 64.7 ± 205.4 | 48.0 ± 129.4 |

| Month 12- all patients^{b}, N | 41 | 39 |
|---|---|---|
| Mean ± SD (nmol/L) | 359.7 ± 113.4 | 287.4 ± 160.9 |
| Changes from baseline | 74.7 ± 195.0 | 50.4 ± 174.9 |

| | | |
|---|---|---|
| ^{a} Includes data from only those patients who completed 12 months of study drug. Patients who completed 12 months of study drug were on drug up to the time of the Final Visit. ^{b} Includes data from the Final Visit for all patients, including both those patients who completed 12 months of study drug, and those who withdrew early. *Normal limits are 138 to 690 nmol/L. | | |

### EXAMPLE 6

### Budesonide MMX® for Add-on Therapy in Patients with Ulcerative Colitis (UC)

To establish the incremental benefit of budesonide MMX when added to current oral therapy, a randomized, double blind, placebo-controlled Phase 3b clinical study of budesonide MMX 9 mg tablets having the composition as in Example 2a, is conducted in patients with mild or moderate active ulcerative colitis (UC) who are not adequately controlled on background oral 5-ASA therapy.

The Phase 3b study evaluates patients with mild to moderate active ulcerative colitis who continue using their current 5-ASA treatment regimen and for an 8 week period add either budesonide MMX 9 mg, or placebo administered once daily. Approximately 500 patients are enrolled, with 250 in each treatment arm. The patients have been on a therapeutic dose of their oral 5-ASA (defined in this study as mesalamine ≥ 2.4 g/day, or equivalent dose of another approved 5-ASA (Table 5)) for a minimum of 6 weeks prior to randomization, and present with signs and symptoms of active, mild to moderate UC (UCDAI score ≥ 4 and ≤ 10 with a mucosal appearance score ≥ 1) in spite of their background therapy. The study compares the two treatment groups over 56 days (8 weeks). Patients remain on the same preparation and dosage strength of their oral 5-ASA for the duration of the study. Eligible patients are randomized to one of the following two treatment arms: 1. Budesonide MMX 9 mg (one tablet); 2. Placebo (tablet indistinguishable from budesonide MMX 9 mg tablet). The assigned study drugs are taken each morning after breakfast. Six visits to the clinical center are scheduled: one at Screening, four during the double-blind treatment period (Day 1, Day 14, Day 28, and Day 56), and one Safety Follow-up Visit 28 days (4 weeks) after the Day 56 visit. At Screening and Visit 5 (Day 56), patients are required to undergo a flexible sigmoidoscopy (or colonoscopy, if clinically indicated) with one photograph and three mucosal biopsies taken from the most severely affected region(s) of the colon visualized during the endoscopy procedure. Patients who are withdrawn early from the study before Day 56 are required to visit the study center as soon as possible so that the final assessments can be conducted.

The primary endpoint of the study is remission at week 8, defined as an Ulcerative Colitis Disease Activity Index (UCDAI) score of less than or equal to 1, with a zero score for rectal bleeding, stool frequency and mucosal appearance.

Patients receiving 9 mg budesonide MMX will experience a higher rate of remission of UC than patients receiving placebo.

**TABLE 5**

| Acceptable Background 5-ASA Formulations and Minimum Required Daily Doses | | |
|---|---|---|
| Brand Name | Generic Name | Minimum Required Daily Dose |
| ASACOL®, ASACOL® HD, LIALDA®, PENTASA®, DELZICOL™ | mesalamine | ≥ 2.4 g |
| AZULFIDINE® | sulfasalazine | ≥ 4.0 g |
| DIPENTUM® | olsalazine | ≥ 2.0 g |
| COLAZAL®, COLAZIDE® | balsalazide | ≥ 6.75 g |

### EXAMPLE 7

### Induction of Remission with Budesonide MMX® (9 mg) Tablets in Patients with Active, Mild to Moderate Ulcerative Colitis: A Multicenter, Open-Label Efficacy and Safety Study

To assess the safety and efficacy of 8 weeks of open-label treatment with oral budesonide MMX 9 mg extended-release tablets having the composition as in Example 2A, in patients with active, mild to moderate UC, who failed to achieve clinical and endoscopic remission in a previous Phase 3, 8-week induction study, patients from a previous Phase 3 study (Parent Study) who completed 8 weeks of induction therapy in any treatment group (budesonide MMX 9 mg; budesonide MMX 6 mg; Asacol® [mesalamine] 2400 mg; or placebo), and who were not in remission were eligible to enroll in the present study to receive an 8 week treatment of open label budesonide MMX 9 mg. Hence, patients who received prior budesonide MMX therapy (9 mg or 6 mg) in the previous Phase 3 study would receive an additional 8 weeks of budesonide MMX 9 mg , therapy for a total duration of 16 weeks.

The primary endpoint was induction of clinical and endoscopic remission, defined by strict criteria with a UCDAI score ≤1 after 8 weeks, with scores of 0 for rectal bleeding and stool frequency, no mucosal friability after colonoscopy, and ≥1-point reduction from baseline in the endoscopic index score. Clinical improvement (≥3 point reduction in UCDAI), endoscopic improvement (≥1 point reduction in the UCDAI mucosal appearance subscore), and symptom resolution (score of 0 for rectal bleeding and stool frequency from UCDAI) were also evaluated at the end of the treatment.

The safety variables were adverse events (AEs), laboratory test results, urinalysis, vital signs, and physical examination findings. In particular, plasma cortisol levels and potential glucocorticoid-related effects were clinically relevant parameters for this evaluation since patients previously randomized to budesonide MMX 6 or 9 mg in the Parent Study would receive budesonide MMX therapy for up to 16 weeks. All endpoints were summarized using descriptive statistics. Results were presented as overall rates of remission, clinical improvement, endoscopic improvement, and symptom resolution.

Sixty patients were administered dosages of 9 mg budesonide MMX. Fifty-two (86.7%) completed the study. Overall rates of clinical and endoscopic remission, clinical improvement, endoscopic improvement, and symptom resolution were 25.0%, 26.7%, 40.0%, and 33.3%, respectively.

Mean morning plasma cortisol (MPC) levels remained within normal limits (138 to 690 nmol/L) regardless of prior therapy (228.1 nmol/L and 167.4 nmol/L at Baseline and Week 8/Final Visit, respectively). Among the 28 patients previously exposed to 8 weeks of budesonide MMX treatment, the additional 8 weeks of open label budesonide MMX 9 mg showed a minimal decrease in MPC relative to baseline (mean Δ= -4.0 nmol/L, SD = 149.47).

Overall frequencies of Treatment Emergent AEs (TEAEs) were similar across treatment groups. The most commonly reported TEAEs (≥5%) were blood cortisol decreased, urinary tract infection, and Cushingoid. Potential glucocorticoid-related effects (Table 6) and Treatment-related AEs were infrequent and occurred to a similar extent by treatment groups (Table 7).

About 25% of patients achieved clinical and endoscopic remission and 33.3% achieved complete symptom resolution with an 8 weeks open label budesonide MMX 9 mg after initial treatment allocation in a placebo controlled trial failed to induce remission. An 8 week course of budesonide MMX 9 mg in patients previously exposed to budesonide MMX (9 mg or 6 mg) treatment does not significantly affect MPC at Week 16/Final Visit. Thus, therapy with budesonide MMX for up to 16 consecutive weeks was well-tolerated.

**TABLE 6**

| | Original Parent Study Treatment Groups | | | |
|---|---|---|---|---|
| | placebo (N= 12) n(%) | budesonide MMX 9 mg* (N= 12) n(%) | budesonide MMX 6 mg* (N= 16) n(%) | Asacol (N= 20) n(%) |
| Overall | 1 (8.3) | 0 (0.0) | 2 (12.5) | 2 (10.0) |
| Moon face | 1 (8.3) | 0 (0.0) | 1 (6.3) | 1 (5.0) |
| Striae Rubrae | 0 (0.0) | 0 (0.0) | 0 (0.0) | 0 (0.0) |
| Flushing | 0 (0.0) | 0 (0.0) | 0 (0.0) | 0 (0.0) |
| Fluid Retention | 0 (0.0) | 0 (0.0) | 1 (6.3) | 0 (0.0) |
| Moon Changes | 0 (0.0) | 0 (0.0) | 0 (0.0) | 0 (0.0) |
| Sleep Changes | 0 (0.0) | 0 (0.0) | 0 (0.0) | 0 (0.0) |
| Insomnia | 0 (0.0) | 0 (0.0) | 0 (0.0) | 0 (0.0) |
| Acne | 1 (8.3) | 0 (0.0) | 0 (0.0) | 0 (0.0) |
| Hirsutism | 0 (0.0) | 0 (0.0) | 0 (0.0) | 0 (0.0) |

**TABLE 7**

| | Original Parent Study Treatment Groups | | | |
|---|---|---|---|---|
| | placebo (N= 12) n (%) | budesonide MMX 9 mg* (N= 12) n(%) | budesonide MMX 6 mg* (N= 16) n(%) | Asacol (N= 20) n (%) |
| Any TEAEs | 8 (66.7) | 6 (50.0) | 7 (43.8) | 12 (60.0) |
| Treatment-related AEs | 2 (16.7) | 1 (8.3) | 2 (12.5) | 3 (12.5) |
| Blood cortisol decrease | 2 (16.7) | 1 (8.3) | 0 (0.0) | 1 (5.0) |
| Cushingoid | 0 (0.0) | 0 (0.0) | 1 (6.3) | 1 (5.0) |
| Pyrexia | 0 (0.0) | 0 (0.0) | 0(0.0) | 1 (5.0) |
| Fluid retention | 0 (0.0) | 0 (0.0) | 1 (6.3) | 0 (0.0) |
| Discontinuations | 0 (0.0) | 0(0.0) | 1 (6.3) | 1 (5.0) |
| due to TEAEs | | | | |

### EXAMPLE 8

### Induction of Remission with Budesonide MMX® (9 mg) Tablets in Patients with Active, Mild to Moderate Ulcerative Colitis

Two similarly-designed, randomized, double-blind, placebo-controlled studies were conducted in a total of 970 adult patients with active, mild to moderate ulcerative colitis (UC) which was defined as an Ulcerative Colitis Disease Activity Index (UCDAI of ≥ 4 and ≤ 10). Eight-hundred ninety-nine of these patients had histology consistent with active UC; this was considered the primary analysis population. UCDAI is a four-component scale (total score of 0 to 12) that encompasses the clinical assessments of stool frequency, rectal bleeding, mucosal appearance and physician's rating of disease activity (score of 0 to 3 for each of the components).

The baseline median UCDAI score in both studies was 7.

In Study 1, 56% of patients were male, and the median age was 42 years. In Study 2, 57% of patients were male, and the median age was 44 years. In Study 1, 50% of patients were Caucasian, 7% were African American, and 34% were Asian. In Study 2, more than 99% were Caucasian.

Both studies compared 9 mg and 6 mg budesonide MMX, having compositions as in Examples 2A and 2B respectively, with placebo and included an active reference arm (a mesalamine 2.4 g in Study 1; and a budesonide* 9 mg not approved for the treatment of UC in Study 2). The primary endpoint was induction of remission after 8 weeks of treatment. Remission was defined as a UCDAI score of ≤ 1, with subscores of 0 for rectal bleeding, stool frequency, and mucosal appearance and with a ≥ 1 point reduction in an endoscopy-only score.

In both studies, 9 mg extended release tablets having the composition as in Example 2A demonstrated superiority to placebo in inducing remission (Table 8).

**TABLE 8**

| Induction of Remission in Studies 1 and 2 | | |
|---|---|---|
| Treatment Group | Study 1 | Study 2 |
| | n/N (%) | n/N (%) |
| 9 mg budesonide | 22/123 (17.9) | 19/109 (17.4) |
| 6 mg budesonide | 16/121 (13.2) | 9/109 (8.3) |
| Reference Arm | 15/124 (12.1) | 13/103 (12.6) |
| Placebo | 9/121 (7.4) | 4/89 (4.5) |
| Treatment difference | 10.4% (2.2%. 18.7%) | 12.9% (4.6%, 21.3%) |
| between 9 mg | | |
| budesonide tablet and Placebo (95% CI)¹ | | |

| | | |
|---|---|---|
| CI = Confidence Interval ¹ p<0.025 for budesonide 9 mg vs. placebo in both Studies 1 and 2 based on the Chi-square test (alpha = 0.025) | | |

The use of the terms "a" and "an" and "the" and similar referents in the context of describing the invention (especially in the context of the following claims) are to be construed to cover both the singular and the plural, unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. For example, if the range 10-15 is disclosed, then 11, 12, 13, and 14 are also disclosed. All methods described herein can be performed in any suitable order unless otherwise indicated herein or otherwise clearly contradicted by context. The use of any and all examples, or exemplary language (e.g., "such as") provided herein, is intended merely to better illuminate the invention.

## Claims

1. A medicament comprising in combination a first and a second composition for simultaneous, separate or sequential use in treating ulcerative colitis in a patient, said first composition comprising 5-aminosalicylic acid, said second composition being in the form of a single tablet comprising:
(1) a tablet core comprising:
a) 9 mg of budesonide;
b) at least one lipophilic excipient;
c) at least one amphiphilic excipient; and
d) at least one hydrophilic excipient; and
(2) a coating on said tablet core, said coating comprising a gastro-resistant film;.
wherein the patient is treated with the first composition previously to the second composition; and wherein said patient has a UCDAI score of greater than or equal to 4 prior to said second composition being administered to said patient.

2. The medicament for use according to claim 1, wherein said patient is experiencing an ulcerative colitis flare prior to said second composition being administered to said patient.

3. The medicament for use according to any one of claims 1 to 2, wherein the second composition is administered for up to 8 weeks.

4. The medicament for use according to any one of claims 1 to 3, wherein after said treatment, said UCDAI score for said patient is less than or equal to 1.

5. A medicament comprising in combination a first and a second composition for use in treating ulcerative colitis in a patient, said first composition comprising 5-aminosalicylic acid, said second composition being in the form of a single tablet comprising:
1) a tablet core comprising:
a) 9 mg of budesonide;
b) at least one lipophilic excipient;
c) at least one amphiphilic excipient; and
d) at least one hydrophilic excipient; and
(2) a coating on said tablet core, said coating comprising a gastro-resistant film;
wherein said second composition is administered to said patient once daily for up to 8 weeks or a portion thereof, wherein said ulcerative colitis in said patient is in remission after said treatment, and wherein said patient has a UCDAI score of greater than or equal to 4 prior to said second composition being administered to said patient.

6. The medicament for use according to claim 5, wherein said second composition is administered to said patient for 8 weeks.

7. The medicament for use according to claim 5, wherein after said treatment, said UCDAI score for said patient is less than or equal to 1.

8. The medicament for use according to claim 5, wherein said patient is experiencing an ulcerative colitis flare prior to said second composition being administered to said patient.

9. The medicament for use according to claim 5, wherein said first and said second compositions are administered to said patient simultaneously.

10. A medicament comprising in combination a first and a second composition for use in treating ulcerative colitis in a patient, said patient having been previously administered a composition comprising 5-aminosalicylic acid, wherein said first composition comprises 5-aminosalicylic acid, said second composition being in the form of a single tablet comprising:
1) a tablet core comprising:
a) 9 mg of budesonide;
b) at least one lipophilic excipient;
c) at least one amphiphilic excipient; and
d) at least one hydrophilic excipient; and
(2) a coating on said tablet core, said coating comprising a gastro-resistant film;
wherein said second composition is administered to said patient once daily for up to 8 weeks or a portion thereof, wherein said ulcerative colitis in said patient is in remission after said treatment, and wherein said patient has a UCDAI score of greater than or equal to 4 prior to said second composition being administered to said patient.

11. The medicament according to claim 10, wherein second composition is administered to said patient for 8 weeks.

12. The medicament according to claim 10, wherein after said treatment, said UCDAI score for said patient is less than or equal to 1.

13. The medicament according to claim 10, wherein said patient is experiencing an ulcerative colitis flare prior to said second composition being administered to said patient.

14. The medicament according to claim 10, wherein said first composition and said second composition are administered to said patient simultaneously.

## Patentansprüche

1. Medikament, das in Kombination eine erste und eine zweite Zusammensetzung zur gleichzeitigen, getrennten oder aufeinanderfolgenden Verwendung bei der Behandlung von Colitis ulcerosa bei einem Patienten umfasst, wobei die erste Zusammensetzung 5-Aminosalicylsäure umfasst, wobei die zweite Zusammensetzung in Form einer einzelnen Tablette vorliegt, die Folgendes umfasst:
(1) einen Tablettenkern, umfassend:
a) 9 mg Budesonid;
b) mindestens einen lipophilen Hilfsstoff;
c) mindestens einen amphiphilen Hilfsstoff; und
d) mindestens einen hydrophilen Hilfsstoff; und
(2) eine Beschichtung auf dem Tablettenkern, wobei die Beschichtung einen gastroresistenten Film umfasst;
wobei der Patient vor der zweiten Zusammensetzung mit der ersten Zusammensetzung behandelt wird; und
wobei der Patient einen UCDAI-Wert größer oder gleich 4 aufweist, bevor dem Patienten die zweite Zusammensetzung verabreicht wird.

2. Medikament zur Verwendung nach Anspruch 1, wobei bei dem Patienten ein Colitis-ulcerosa-Schub auftritt, bevor dem Patienten die zweite Zusammensetzung verabreicht wird.

3. Medikament zur Verwendung nach einem der Ansprüche 1 bis 2, wobei die zweite Zusammensetzung für bis zu 8 Wochen verabreicht wird.

4. Medikament zur Verwendung nach einem der Ansprüche 1 bis 3, wobei nach der Behandlung der UCDAI-Wert des Patienten kleiner oder gleich 1 ist.

5. Medikament, das in Kombination eine erste und eine zweite Zusammensetzung zur Verwendung bei der Behandlung von Colitis ulcerosa bei einem Patienten umfasst, wobei die erste Zusammensetzung 5-Aminosalicylsäure umfasst, wobei die zweite Zusammensetzung in Form einer einzelnen Tablette vorliegt, die Folgendes umfasst:
(1) einen Tablettenkern, umfassend:
a) 9 mg Budesonid;
b) mindestens einen lipophilen Hilfsstoff;
c) mindestens einen amphiphilen Hilfsstoff; und
d) mindestens einen hydrophilen Hilfsstoff; und
(2) eine Beschichtung auf dem Tablettenkern, wobei die Beschichtung einen gastroresistenten Film umfasst;
wobei die zweite Zusammensetzung dem Patienten für bis zu 8 Wochen oder einen Teil davon einmal pro Tag verabreicht wird, wobei sich die Colitis ulcerosa nach der Behandlung in Remission befindet und wobei der Patient einen UCDAI-Wert größer oder gleich 4 aufweist, bevor dem Patienten die zweite Zusammensetzung verabreicht wird.

6. Medikament zur Verwendung nach Anspruch 5, wobei die zweite Zusammensetzung dem Patienten für 8 Wochen verabreicht wird.

7. Medikament zur Verwendung nach Anspruch 5, wobei nach der Behandlung der UCDAI-Wert des Patienten kleiner oder gleich 1 ist.

8. Medikament zur Verwendung nach Anspruch 5, wobei bei dem Patienten ein Colitis-ulcerosa-Schub auftritt, bevor dem Patienten die zweite Zusammensetzung verabreicht wird.

9. Medikament zur Verwendung nach Anspruch 5, wobei dem Patienten die erste und die zweite Zusammensetzung gleichzeitig verabreicht werden.

10. Medikament, das in Kombination eine erste und eine zweite Zusammensetzung zur Verwendung bei der Behandlung von Colitis ulcerosa bei einem Patienten umfasst, wobei dem Patienten zuvor eine 5-Aminosalicylsäure umfassende Zusammensetzung verabreicht wurde, wobei die erste Zusammensetzung 5-Aminosalicylsäure umfasst, wobei die zweite Zusammensetzung in Form einer einzelnen Tablette vorliegt, die Folgendes umfasst:
(1) einen Tablettenkern, umfassend:
a) 9 mg Budesonid;
b) mindestens einen lipophilen Hilfsstoff;
c) mindestens einen amphiphilen Hilfsstoff; und
d) mindestens einen hydrophilen Hilfsstoff; und
(2) eine Beschichtung auf dem Tablettenkern, wobei die Beschichtung einen gastroresistenten Film umfasst;
wobei die zweite Zusammensetzung dem Patienten für bis zu 8 Wochen oder einen Teil davon einmal pro Tag verabreicht wird, wobei sich die Colitis ulcerosa bei dem Patienten nach der Behandlung in Remission befindet und wobei der Patient einen UCDAI-Wert größer oder gleich 4 aufweist, bevor dem Patienten die zweite Zusammensetzung verabreicht wird.

11. Medikament nach Anspruch 10, wobei die zweite Zusammensetzung dem Patienten für 8 Wochen verabreicht wird.

12. Medikament nach Anspruch 10, wobei nach der Behandlung der UCDAI-Wert des Patienten kleiner oder gleich 1 ist.

13. Medikament nach Anspruch 10, wobei bei dem Patienten ein Colitis-ulcerosa-Schub auftritt, bevor dem Patienten die zweite Zusammensetzung verabreicht wird.

14. Medikament nach Anspruch 10, wobei dem Patienten die erste Zusammensetzung und die zweite Zusammensetzung gleichzeitig verabreicht werden.

## Revendications

1. Médicament comprenant en combinaison une première et une seconde composition pour une utilisation simultanée, séparée ou séquentielle dans le traitement de la colite ulcéreuse chez un patient, ladite première composition comprenant de l'acide 5-aminosalicylique, ladite seconde composition se présentant sous la forme d'un seul comprimé comprenant :
(1) un noyau de comprimé comprenant :
a) 9 mg de budésonide ;
b) au moins un excipient lipophile ;
c) au moins un excipient amphiphile ; et
d) au moins un excipient hydrophile ; et
(2) un enrobage sur ledit noyau de comprimé, ledit enrobage comprenant un film gastro-résistant ;
dans lequel le patient est traité avec la première composition préalablement à la seconde composition ; et
dans lequel ledit patient a un score UCDAI supérieur ou égal à 4 avant l'administration de ladite seconde composition audit patient.

2. Médicament à utiliser selon la revendication 1, dans lequel ledit patient subit une poussée de colite ulcéreuse avant l'administration de ladite seconde composition audit patient.

3. Médicament à utiliser selon l'une quelconque des revendications 1 à 2, dans lequel la seconde composition est administrée jusqu'à 8 semaines.

4. Médicament à utiliser selon l'une quelconque des revendications 1 à 3, dans lequel, après ledit traitement, ledit score UCDAI destiné audit patient est inférieur ou égal à 1.

5. Médicament comprenant en combinaison une première et une seconde composition destinée à être utilisée dans le traitement de la colite ulcéreuse chez un patient, ladite première composition comprenant de l'acide 5-aminosalicylique, ladite seconde composition se présentant sous la forme d'un seul comprimé comprenant :
1) un noyau de comprimé comprenant :
a) 9 mg de budésonide ;
b) au moins un excipient lipophile ;
c) au moins un excipient amphiphile ; et
d) au moins un excipient hydrophile ; et
(2) un enrobage sur ledit noyau de comprimé, ledit enrobage comprenant un film gastro-résistant ;
dans lequel ladite seconde composition est administrée audit patient une fois par jour jusqu'à 8 semaines ou une partie de cette période, dans lequel ladite colite ulcéreuse chez ledit patient est en rémission après ledit traitement, et dans lequel ledit patient a un score UCDAI supérieur ou égal à 4 avant l'administration de ladite seconde composition audit patient.

6. Médicament à utiliser selon la revendication 5, dans lequel ladite seconde composition est administrée audit patient pendant 8 semaines.

7. Médicament à utiliser selon la revendication 5, dans lequel, après ledit traitement, ledit score UCDAI destiné audit patient est inférieur ou égal à 1.

8. Médicament à utiliser selon la revendication 5, dans lequel ledit patient subit une poussée de colite ulcéreuse avant l'administration de ladite seconde composition audit patient.

9. Médicament à utiliser selon la revendication 5, dans lequel lesdites première et seconde compositions sont administrées audit patient simultanément.

10. Médicament comprenant en combinaison une première et une seconde composition destinées à être utilisées dans le traitement de la colite ulcéreuse chez un patient, ledit patient ayant préalablement reçu une composition comprenant de l'acide 5-aminosalicylique, dans lequel ladite première composition comprend de l'acide 5-aminosalicylique, ladite seconde composition se présentant sous la forme d'un seul comprimé comprenant :
1) un noyau de comprimé comprenant :
a) 9 mg de budésonide ;
b) au moins un excipient lipophile ;
c) au moins un excipient amphiphile ; et
d) au moins un excipient hydrophile ; et
(2) un enrobage sur ledit noyau de comprimé, ledit enrobage comprenant un film gastro-résistant ;
dans lequel ladite seconde composition est administrée audit patient une fois par jour jusqu'à 8 semaines ou une partie de cette période, dans lequel ladite colite ulcéreuse chez ledit patient est en rémission après ledit traitement, et dans lequel ledit patient a un score UCDAI supérieur ou égal à 4 avant que ladite seconde composition n'est administrée audit patient.

11. Médicament selon la revendication 10, dans lequel la seconde composition est administrée audit patient pendant 8 semaines.

12. Médicament selon la revendication 10, dans lequel, après ledit traitement, ledit score UCDAI destiné audit patient est inférieur ou égal à 1.

13. Médicament selon la revendication 10, dans lequel ledit patient subit une poussée de colite ulcéreuse avant l'administration de ladite seconde composition audit patient.

14. Médicament selon la revendication 10, dans lequel ladite première composition et ladite seconde composition sont administrées audit patient simultanément.
